Europäisches Patentamt

European Patent Office

Office européen des brevets

(19)

(11) Numéro de publication : **0 480 981 B1**

(12)

# FASCICULE DE BREVET EUROPEEN

(45) Date de publication du fascicule du brevet :
20.10.93 Bulletin 93/42

(51) Int. Cl.⁵ : **A61K 39/39, A61K 9/113**

(21) Numéro de dépôt : **90910772.4**

(22) Date de dépôt : **29.06.90**

(86) Numéro de dépôt international :
**PCT/FR90/00484**

(87) Numéro de publication internationale :
**WO 91/00106 10.01.91 Gazette 91/02**

(54) EMULSIONS MULTIPHASIQUES INJECTABLES.

(30) Priorité : **03.07.89 FR 8908917**

(43) Date de publication de la demande :
**22.04.92 Bulletin 92/17**

(45) Mention de la délivrance du brevet :
**20.10.93 Bulletin 93/42**

(84) Etats contractants désignés :
**AT BE CH DE DK ES FR GB IT LI LU NL SE**

(56) Documents cités :
EP-A- 0 174 377
FR-A- 1 603 312
GB-A- 1 408 437
CHEMIST & DRUGGIST, vol. 213, 26 January
1980; D. WHITEHILL, pp. 130, 132, 135

(73) Titulaire : **S.E.P.P.I.C., SOCIETE
D'EXPLOITATION DE PRODUITS POUR LES
INDUSTRIES CHIMIQUES
75, quai d'Orsay
F-75321 Paris Cédex 07 (FR)**

(72) Inventeur : **BRANCQ, Bernard
2, rue d'Armenonville
F-78150 Le Chesnay (FR)**
Inventeur : **TROUVE, Gérard
134, chemin du Rozé
F-81100 Castres (FR)**

(74) Mandataire : **Portal, Gérard et al
Cabinet Beau de Loménie 158, rue de
l'Université
F-75340 Paris Cédex 07 (FR)**

## Description

La présente invention concerne des émulsions multi-phasiques utilisables pour l'administration de principes actifs ou d'antigènes par voie injectable ; ces émulsions constituent de nouveaux véhicules bien tolérés qui permettent une libération progressive des principes actifs ou des antigènes.

Une application particulière de la présente invention concerne des vaccins multiphasiques, les adjuvants d'immunité qu'ils contiennent et leur procédé de préparation.

Les adjuvants d'immunité sont des produits qui augmentent les réactions du système immunitaire lorsqu'ils sont administrés en présence d'antigènes d'origine virale, bactérienne ou synthétique : ils provoquent L'apparition massive de macrophages au site d'injection, dans les nodules lymphatiques, accroissent la production d'immunoglobulines spécifiques (anticorps) et stimulent de nombreuses cellules impliquées dans les mécanismes de défense immunitaire.

Parmi ces adjuvants, il est reconnu que ceux résultant de l'association d'une huile minérale et d'un ester de mannitol, contenant ou non une mycobactérie tuée, connus sous le nom d'adjuvants de Freund (IFA), sont les plus efficaces.

Les vaccins réalisés par mélange en part égale d'adjuvant de Freund et de milieu antigénique restent la référence dans le monde entier pour des études immunologiques de laboratoire. Ils se présentent sous forme d'émulsions à phase continue huileuse (E/H), très visqueuses (de l'ordre de 5000 mPa.s à 20°C), donc difficilement injectables et de plus peu stables : des déphasages sont observés au bout de quelques jours.

Ces vaccins sont très mal tolérés et provoquent, au niveau du site d'injection chez les animaux, des réactions locales très importantes avec oedèmes, abcès et nécroses, inacceptables par les autorités sanitaires et qui rendent la viande des animaux de boucherie impropre à la consommation.

Chez les humains, des vaccins anti-diphtériques réalisés avec de l'adjuvant de Freund complet ont provoqué des intolérances sévères, voire même des avortements.

Afin d'améliorer l'injectabilité de ces vaccins, un procédé consistant à les redisperser dans une phase aqueuse contenant un agent tensioactif hydrophile (Polysorbate 80) a été décrit par HERBERT (Thé LANCET 16, 771, 1965).

Les emulsions doubles obtenues sont fluides, mais leur stabilité n'est que de quelques jours.

Le tensioactif hydrophile utilisé est toxique vis-à-vis des cellules puisqu'il est largement utilisé par ailleurs en biologie pour délipider des membranes cellulaires.

La préparation de ce type d'émulsions multiples est délicate : elle dépend non seulement de la composition des phases mais aussi du mode opératoire en utilisant le mode d'agitation par ultrason décrit par HERBERT, des auteurs ont obtenu une émulsion multiple par sonication pendant 10s, alors que la même formule est de type E/H si la sonication est poursuivie 30s.

Une aussi grande variabilité sur les paramètres du procédé, qui, de plus, nécessite deux étapes, est difficilement acceptable à l'échelle industrielle.

Du point de vue immunitaire, les vaccins obtenus par ce procédé se sont avérés moins Performants que des vaccins de type E/H. (ANDERSON. Res. Vet. Sci 12, 18, (1971) HERBERT. Immuno-logical standardization. Symposium series 6, 29, (1967)).

Il serait donc intéressant de disposer d'émulsions fluides, facilement injectables, stables, de type E/H/E à température ambiante qui pourraient se transformer, à la température du corps humain ou animal, en une préparation de type E/H plus efficace.

Cette transformation, appelée inversion de phase, a été largement étudiée par SHINODA et son équipe. La température à laquelle se produit le passage de la forme E/H à la forme E/H/E est la température d'inversion de phase. Elle est déterminée par suivi de la conductivité électrique d'une émulsion fabriquée à chaud et refroidie dans un bain de glace.

L'inversion de phase se produit avec de nombreux tensioactifs non ioniques mais a été étudiée surtout sur des produits de type alkyl phénol éthoxylé, incompatibles avec un usage médical ou vétérinaire.

Les émulsions, objets de la présente invention, sont fabriquées en une seule opération, à partir d'émulgateurs pharmaceutiquement acceptables qui, dissous dans une huile injectable, forment une phase homogène limpide et ont des points d'inversion voisins des températures des corps animaux ou humains. Il a de plus été observé, de façon tout à fait inattendue, que ces vaccins sont très bien tolérés et ne provoquent pas de réactions locales, abcès ou nécroses comme le font les vaccins de type E/H réalisés à partir de la même huile.

Caractérisation des émulsions selon l'invention

Les émulsions obtenues sont caractérisées par les propriétés suivantes :

- fluidité : mesurées avec un appareil BROOKFIELD à 20°C, les viscosités sont toujours inférieures à 300 mPas, généralement inférieures à 100 mPas
- injectabilité : les émulsions s'écoulent facilement hors d'une seringue de 10 ml munie d'une aiguille de diamètre 0,8 mm et soumise à une force constante de 3,3 kg.

Le temps nécessaire à l'écoulement de 10 ml d'émulsion est comparable à celui d'une formule H/E et nettement plus faible que celui d'une émulsion avec l'adjuvant de Freund.

| Emulsion | Temps d'écoulement (s) |
|---|---|
| selon l'invention (E/H/E) | 9 |
| H/E | 6,5 |
| contenant IFA (E/H) | 59 |

- conductivité : elle détermine le caractère de la phase continue de l'émulsion. Elle est généralement comprise entre 0,1 et 10 millisiemens à froid, entre 0,05 et 5 microsiemens à chaud
- dispersibilité dans l'eau : les émulsions obtenues selon l'invention se dispersent facilement dans l'eau avec laquelle elles forment une solution laiteuse avec une pellicule huileuse
- aspect microscopique : les émulsions selon l'invention ont des tailles de gouttes huileuses inférieures ou voisines de 1 μm.

Les microgouttes de phase aqueuse dans ces gouttes huileuses sont invisibles au microscope optique. Ces tailles de particules, très faibles comparativement à celles des formules type HERBERT, expliquent la bonne stabilité des émulsions de vaccins de l'invention :

-stabilité : les émulsions selon l'invention sont stables au moins 12 mois à 4°C.

Pour déterminer si un mélange d'huile, d'émulgateurs et de milieu antigénique mis sous agitation, selon le mode opératoire de l'invention, a permis la réalisation d'une émulsion multiple, il suffit de faire tomber quelques gouttes de la préparation obtenue dans un récipient d'eau et d'agiter doucement avec une spatule.

Une émulsion du type eau dans l'huile donne des gouttes blanches distinctes qui vont rapidement se regrouper et surnager à la surface de l'eau.

Une émulsion du type huile dans l'eau donne un lait de couleur et d'aspect homogènes.

Une émulsion multiple donne à la fois un lait homogène et des gouttelettes surnageant à la surface de l'eau.

Caractérisation des huiles contenues dans les émulsions injectables

Les huiles doivent être dépourvues de toxicité et donner des émulsions fluides au stockage à 4°C.

Elles sont choisies parmi les huiles minérales, végétales ou animales connues pour leur faible toxicité. On choisira des huiles minérales à chaînes linéaires ayant un nombre d'atomes de carbone supérieur à 16 et exemptes de composés aromatiques.

Des exemples connus sont le MARCOL 52 (produit par ESSO France) ou le DRAKEOL 6VR (produit par PENRECO USA).

On peut également utiliser des hydrocarbures de synthèse tels que le polyisobutène ou le polyisoprène.

Pour les huiles végétales, on choisira des huiles insaturées de type oléique qui sont biodégradables et connues pour leur pouvoir immunogène, par exemple les huiles d'arachide, d'olive, de sésame, de soja, de germe de blé, de jojoba, etc...

Pour les huiles animales, les mêmes critères de tolérance et d'efficacité immunologiques permettent d'utiliser par exemple du squalène, du squalane, de l'huile de spermaceti.

Des esters d'acides gras ayant au moins 14 atomes de carbone et d'alcool, de préférence ramifiés, liquides à la température de stockage peuvent également être utilisés.

Ainsi, selon une caractéristique particulière de l'invention, l'huile utilisée est une huile minérale ou un hydrocarbure de synthèse, liquide à 4°C et ayant une viscosité inférieure à 100 mPa.s à 40°C.

Selon une autre caractéristique de l'invention, l'huile utilisée est une huile ou une cire liquide métabolisable d'origine végétale, vierge ou raffinée.

Selon encore une autre caractéristique de l'invention, l'huile utilisée est une huile d'origine animale, en particulier de poisson.

L'huile utilisée selon l'invention peut être également un mélange de deux ou plusieurs des huiles décrites ci-dessus.

Caractérisation des émulgateurs

Ce sont des produits non ioniques dépourvus de toxicité notable, utilisables par voie injectable, qui peuvent être choisis dans les classes chimiques ci-dessous, citées à titre d'exemples non limitatifs :
- esters ou éthers d'acide gras et de sucre (SORBITOL, MANNITOL, SACCHAROSE, GLUCOSE...)
- esters d'acide gras et de glycérol ou de polyol
- dérivés hydrophiles de ces esters obtenus par greffage de fonctions alcool, éther-oxyde, carboxylique, amine, amide...
- lécithines
- acides ou alcools gras condensés avec de L'oxyde d'éthylène et ou de propylène.

Les chaînes grasses des émulgateurs utilises auront de 8 à 22 atomes de carbone, de préférence de 14 à 20 atomes de carbone. Les chaînes liquides sont préférées.

Les alcools et acides oléique, ricinoléique, linoléique, isostéarique, cétostéarique et leurs dérivés sont des composés de choix.

Les émulgateurs de la famille des oléates de mannitol sont particulièrement intéressants du fait de leur bonne innocuité et de leur aptitude à former des émulsions multiphasiques très stables. Des dérivés d'oléate de mannitol obtenus par greffage de fonctions hydrophiles telles que, par exemple, des fonctions amine, amide, éthoxy, alcool, polyol, carboxylique... peuvent être avantageusement utilisés.

Des adjuvants d'immunité prêts à l'emploi comprenant l'huile et les émulgateurs et se présentant sous forme d'un liquide huileux limpide, stable et homogène constituent le mode de mise en oeuvre préféré des émulgateurs utilisés dans l'invention.

Procédé pour l'obtention de vaccins multiphases de type E/H/E

Il est important pour obtenir des vaccins très stables de les fabriquer en suivant la procédure suivante :
- porter la phase huileuse (huile et émulgateurs) d'une part et la phase aqueuse contenant les antigènes ou les principes actifs d'autre part à la même température choisie entre 20 et 40°C. Une température de 30°C est souvent optimale
- verser la phase aqueuse dans la phase huileuse sous agitation modérée non cisaillante et agiter jusqu'à retour à la température ambiante.

Des formulations stables plus d'un an à 4°C et à température ambiante sont obtenues par ce procédé.

Exemple 1

On réalise un vaccin ayant la composition suivante :

```
Adjuvant huileux                              50 %
Solution tampon d'albumine  bovine            50 %
à 100 µg/ml
```

La composition de l'adjuvant huileux est choisie pour que le point d'inversion de l'emulsion soit de 35°C environ.

Les caractéristiques de cet adjuvant et du vaccin correspondant sont mentionnées dans le tableau 1 - 1.

Ce vaccin est injecté par voie sous-cutanée à des souris SWISS. L'évolution du taux d'anticorps (moyenne sur 10 souris) déterminée par une technique ELISA est donnée dans le tableau 1 - 2.

A titre de comparaison sont mentionnées également dans ce tableau les évolutions des taux d'anticorps observés pour un vaccin fabriqué avec de l'adjuvant de Freund incomplet (IFA) et pour une solution tampon d'albumine sans adjuvant d'immunité.

Le vaccin décrit dans cet exemple permet d'augmenter de façon significative et durable le taux d'anticorps chez la souris comparativement à un vaccin non adjuvé, sans toutefois atteindre les performances du vaccin contenant l'IFA.

TABLEAU 1 - 1

CARACTERISTIQUES PHYSICO-CHIMIQUES DU VACCIN BSA

Caractéristiques de l'adjuvant

| | |
|---|---|
| ASPECT | huile limpide de couleur jaune paille |
| COMPOSITION | huile minérale fluide  86 % |
| | ester oléique d'anhydro-  14 % |
| | mannitol et de PEG * 500 |
| Indice d'acide | 0,15 |
| Indice d'hydroxyle | 18 |
| Indice de saponification | 15 |
| Indice de réfraction | 1,461 |
| Viscosité | 25 mPas |

* PÉG : polyéthylèneglycol

TABLEAU 1 - 1 (suite)

Caractéristiques du vaccin (à 20°C)

| | |
|---|---|
| Type | E/H/E |
| Taille de particule | <1 µm |
| Conductivité | 1,0 mS |
| Viscosité | 50 mPas |
| Stabilité 4°C | > 12 mois. |

TABLEAU  1 - 2

TAUX  D'ANTICORPS  ANTI  BSA  (EXPRIME EN INVERSE  DE  LA  DILUTION

NECESSAIRE POUR OBTENIR UNE DENSITE OPTIQUE EGALE A 1)

| JOURS APRES VACCINATION | 14 | 28 | 56 | 125 |
|---|---|---|---|---|
| VACCIN SELON EXEMPLE 1 | 95 | 1295 | 2785 | 2225 |
| VACCIN SUR IFA | 475 | 7560 | 17280 | 25600 |
| ALBUMINE SANS ADJUVANT | 60 | 157 | 102 | 105 |

Exemple 2

Un vaccin contre la grippe est testé chez la souris SWISS. Il a pour composition :

adjuvant  huileux  (huile  minérale contenant  des  esters
oléiques  de PEG 500 et                   :   47 %
d'anhydromannitol)
milieu antigénique                        :   53 %

Le milieu antigénique est constitué d'un virus Influenza A/PR8 inactivé au formol titrant 800 unités hémag-glutinantes/ml.

L'adjuvant et le vaccin ont sensiblement les mêmes caractéristiques physico-chimiques que celles de l'exemple 1. Le point d'inversion du vaccin est 37°C.

La vaccination est effectuée au moyen d'une injection unique de 0,2 ml par voie sous-cutanée.

Le tableau 2 donne les résultats d'un dosage d'anticorps (exprimé en log2 des dilutions). On peut y constater que le vaccin multiphasique est aussi performant que le vaccin correspondant sur IFA et nettement plus efficace que le vaccin témoin non adjuvé.

## TABLEAU 2

### PERFORMANCE D'UN VACCIN GRIPPAL MULTIPHASIQUE

| VACCIN | TAUX D'ANTICORPS 42 j après vaccination |
|---|---|
| multiphasique | 4,3 |
| adjuvé IFA | 4,9 |
| non adjuvé | 3,1 |

### Exemple 3

Deux vaccins contre la maladie d'AUJESZKY ont été formulés à partir du même milieu antigénique. L'un est un vaccin huileux classique de type E/H (3A), l'autre est un vaccin multiphasique E/H/E selon l'invention (3B).

Les adjuvants contenus dans ces deux vaccins sont basés sur une huile de synthèse liquide, obtenue par polymérisation de l'isobutylène. Le vaccin multiphasique contient un système émulgateur lui conférant un point d'inversion de 33°C environ.

Les caractéristiques des vaccins et de leurs adjuvants sont données dans le tableau 3 - 1.

TABLEAU 3 - 1

| FORMULE | 3A | % | 3B | % |
|---|---|---|---|---|
| **CARACTERISTIQUES DE L'ADJUVANT** | | | | |
| Aspect | limpide, jaune clair | | limpide, jaune clair | |
| Huile | polyisobutène | 88 % | polyisobutène | 85 % |
| Emulgateur | monooléate de mannitol | 12 % | monooléate de mannitol | 7,5 % |
| | | | PEG 10 oléate | 7,5 % |
| Indice d'acide | 0,12 | | 0,15 | |
| Viscosité 20°C | 50 mPas | | 60 mPas | |
| **CARACTERISTIQUES DU VACCIN A 20°C** | | | | |
| Viscosité | 100 mPas | | 88 mPas | |
| Taille de gouttes | 1 à 5 µm | | 1 µm | |
| Conductivité | 0,15 µS | | 3,9 mS | |
| Type | E/H | | E/H/E | |
| Stabilité 4°C | > 12 mois | | > 12 mois | |

Deux lots de chacun 6 porcs charcutiers placés en élevage industriel ont été vaccinés. La vaccination a

8

été effectuée par injection intramusculaire de 2 ml de vaccin suivie d'un rappel à 15 jours. Les taux d'anticorps ont été estimés par séro-neutralisation, 59 jours après la première injection.

Le tableau 3 - 2 montre clairement l'intérêt du vaccin multiphasique 3B qui ne provoque que peu de réactions d'intolérance (nécrose, suppuration, fibrose, atrophie musculaire), tout en donnant une réponse immunologique positive comme l'attestent les résultats de sérologie et la présence de granulomes macrophagiques.

## TABLEAU 3 - 2

### RESULTATS DE VACCINATION

| VACCIN | 3A | 3B |
|---|---|---|
| SEROLOGIE (hémagglutination) | | |
| taux anticorps (10g2) | 2,6 | 1,3 |
| HISTOLOGIE * | | |
| nécrose | 1/6 | 0/6 |
| suppuration | 3/6 | 0/6 |
| fibrose | 6/6 | 3/6 |
| atrophie musculaire grave | 2/6 | 1/6 |
| granulomes macrophagiques | 4/6 | 6/6 |

* fréquence des lésions observées.

## EXEMPLE 4

Cette expérience met à nouveau en évidence la bonne tolérance d'un vaccin multiphasique selon l'invention comparativement à un vaccin huileux classique de type E/H formulé avec la même huile minérale.

Les caractéristiques des deux adjuvants huileux et des deux vaccins obtenus sont données dans le tableau 4 - 1.

Deux lots de 5 porcs charcutiers chacun placés en station d'élevage ont été vaccinés contre la maladie d'AUJESZKY.

Deux ml de vaccin ont été administres par voie intra-musculaire à chaque animal ; un rappel a été pratiqué de la même façon 18 jours après la première vaccination. Une épreuve de résistance par pulvérisation intra-nasale de 4 ml d'une suspension virale infectieuse a été effectuée 32 jours après vaccination. Le tableau 4 - 2 résume les résultats.

Les évolutions pondérables des deux groupes d'animaux après épreuve sont comparables et nettement supérieures à celles des témoins vaccinés non adjuvés. Les taux d'anticorps (exprimés en inverse de dilution) ne sont pas significativement différents.

Par contre, la tolérance du vaccin multiphasique 4B est bien meilleure aucun abcès nécrotique ni aucune réaction locale inacceptable par les autorités sanitaires n'ont été observés.

Ceci est à rapprocher des élévations de températures modérées observées avec ce vaccin lors des 2 vac-

cinations.

EP 0 480 981 B1

TABLEAU 4 - 1
CARACTERISTIQUES DES VACCINS CONTRE LA MALADIE D'AUJESZKY

| FORMULE | 4A | % | 4B | % |
|---|---|---|---|---|
| **CARACTERISTIQUES DE L'ADJUVANT** | | | | |
| Huile | huile minérale fluide | 89 % | huile minérale fluide | 85 % |
| Emulgateur | ester d'acide oléique et de mannitol | 11 % | ester oléique de mannitol et de PEG 500 | 15 % |
| Indice d'hydroxyle | 12 | | | |
| Indice d'acide | 0,11 | | 0,2 | |
| Indice de saponification | 14 | | 17 | |
| Indice de réfraction ($25^{\circ}$C) | 1,459 | | 1,460 | |
| Viscosité ($20^{\circ}$C) | 40 mPas | | 20 mPas | |
| **CARACTERISTIQUES DU VACCIN** | | | | |
| Type | E/H | | E/H/E | |
| Viscosité ($20^{\circ}$C) | 25 mPas | | 112 mPas | |
| Aspect microscopique | gouttes env. 1μm | | gouttes <1 μm | |
| Conductivité ($20^{\circ}$C) | 0,28 μS | | 1,3 mS | |
| Stabilité à $4^{\circ}$C | > 12 mois | | > 12 mois | |
| Titre antigénique | 10 9 DCP 50/ml | | 10 9 DCP 50/ml | |

EP 0 480 981 B1

TABLEAU 4 - 2
RESULTATS DE LA VACCINATION CHEZ LE PORC

| VACCIN | 4A | 4B |
|---|---|---|
| HYPERTHERMIE | | |
| à la vaccination | + | - |
| au rappel | +++ | + |
| à l'épreuve | + | ++ |
| EVOLUTION PONDERALE MOYENNE % | | |
| (pendant les 7 j après épreuve) | + 0,17 | + 0,15 |
| NB. Vaccin non adjuvé - 1,08 | | |
| SEROLOGIE | | |
| taux anticorps | 22,4 | 13,6 |
| écart type | 8,8 | 11,2 |
| REACTIONS LOCALES | | |
| abcès - nécroses | 4/4 | 0/5 |
| suppuration | 1/4 | 0/5 |
| fibrose | 3/4 | 2/5 |
| atrophie musculaire | 3/4 | 5/5 |
| granulomes macrophagiques | 3/4 | 2/5 |

TABLEAU 5

| FORMULE | 32 A | % | 3408 | % | 26 K | % |
|---|---|---|---|---|---|---|
| **CARACTERISTIQUES DE L'ADJUVANT** | | | | | | |
| Huile | minérale fluide | 85 % | squalane | 85 % | minérale fluide | 88 % |
| Emulgateur | lécithine monooléate PEG | 7,8 % / 400 | ester oléique de mannitol et PEG | 400 / 15 % | ester oléique de mannitol et PEG | 12 % |
| Aspect | limpide jaune | | limpide jaune clair | | limpide jaune clair | |
| Viscosité 20°C | 35 mPas | 7,2 % | | | 30 mPas | |
| **CARACTERISTIQUES DU VACCIN** | | | | | | |
| Type | multiphasique | | multiphasique | | multiphasique | |
| % adjuvant | 50 | | 50 | | 70 | |
| Viscosité | 30 mPas | | 100 mPas | | 150 mPas | |
| Conductivité | 3 mS | | 2 mS | | 1,8 mS | |
| Aspect microscopique (taille gouttes) | < 1 µm | | 1 µm | | 1 µm | |

TABLEAU 5 (suite)

| FORMULE | GTAF 56 | % | V 7401 - 1 | % | V 7401 - 2 | % |
|---|---|---|---|---|---|---|
| CARACTERISTIQUES DE L'ADJUVANT | | | | | | |
| Huile | huile d'arachide | 92 % | huile minérale + arachide (1:1) | 84 % | huile minérale + arachide (1:1) | 87 % |
| Emulgateur | copolymère OE/OP | 8 % | ester d'acide oléique et de mannitol | 16 % | ester d'acide oléique et de man-nitol+lécithine | 13 % |
| Aspect | jaune clair | | limpide jaune paille | | limpide jaune | |
| Viscosité 20°C | 50 mPas | | 45 mPas | | 50 mPas | |
| CARACTERISTIQUES DU VACCIN | | | | | | |
| Type | multiphasique | | multiphasique | | multiphasique | |
| % adjuvant | 65 | | 64 | | 60 | |
| Viscosité | 1800 mPas | | 450 mPas | | 100 mPas | |
| Conductivité | 1,9 mS | | 2 mS | | 2 mS | |
| Aspect micro-scopique (taille gouttes) | 1 μm | | 1 μm | | 1 μm | |

EP 0 480 981 B1

## Exemple 5

On décrit dans cet exemple des émulsions placebos et des adjuvants correspondants (tableau 5) qui ont tous pour caractéristiques :
- faible viscosité
- bonne injectabilité
- inversion vers 30 - 40°C
- stabilité correcte au stockage à 4°C

Ces exemples montrent que l'on peut réaliser des vaccins selon l'invention à partir de différentes huiles utilisables par voie injectable (minérale, végétale, squalane...) et de leurs mélanges, ainsi que différents types d'émulgateurs à condition que leur concentration soit adaptée. La quantité de phase aqueuse contenant les principes actifs peut également varier dans une large proportion.

Il a cependant été remarqué que les caractéristiques des émulsions obtenues, notamment viscosité, stabilité et point d'inversion, sont très sensibles à la composition des adjuvants et à l'origine des huiles utilisées.

## Exemple 6

Un test de tolérance à l'injection intrapéritonéale de 0,25 ml de produit a été décrit par S.S BERLIN (Annals of Allergy 20, 473 (1962)). Les vaccins selon l'invention passent tous les normes de ce test (pas de mort, de péritonite, évolution pondérale comparable aux témoins).

Des tests conformes aux protocoles décrits dans la pharmacopée européenne ont montré qu'il n'existait pas de toxicité anormale dans des vaccins placebos selon l'invention, en particulier pour ceux formulés à partir d'esters oléiques de mannitol et de PEG, et d'huile minérale fluide ou d'huile synthétique polyisobutylène.

## Exemple 7

Cet exemple illustre l'importance du procédé de fabrication sur les propriétés physico-chimiques des vaccins multiphasiques, notamment la température de fabrication.

Les formules étudiées ont pour composition :

```
Adjuvant huileux (huile minérale 85 %,  esters oléiques de
                    mannitol et PEG 15 %)        94 g
     Milieu antigénique placebo                 106 g
```

Mode opératoire 1 - Les deux phases sont portées à 30°C et agitées ensembles 10 min par un agitateur YSTRAL tournant à 2500 tr/min environ.

Mode opératoire 2 - La phase huileuse est chauffée à 40°C, la phase aqueuse est maintenue à température ambiante.

Aspect microscopique des vaccins obtenus :

| mode opératoire 1 | mode opératoire 2 |
|---|---|
| gouttes très fines (< 1µm) - homogènes | gouttes pouvant aller jusqu'à 50µm hétérogène |

**Revendications**

1. Emulsions injectables multiphasiques de type E/H/E/ utilisables comme vaccins, ou véhicules de principes actifs en médecine vétérinaire ou humaine, pharmaceutiquement acceptables pour chacun de Leurs composants, stables au moins 12 mois au stockage à 4°C, de viscosité inférieure à 300 mPas et comprenant au moins les constituants suivants :
   - un adjuvant huileux composé de :
     . une phase huileuse immiscible à l'eau représentant 20 à 70 % en poids de la formule ;
     . un système émulgateur choisi de façon telle que le point d'inversion de l'émulsion obtenue soit compris entre 25 et 45° et représentant de 2 à 10 % en poids de la formule ;
   - une phase aqueuse contenant les antigènes ou les principes actifs et représentant de 20 à 78 % en poids de la formule et obtenue en une seule opération de mélange.

2. Composition selon la revendication 1, dans laquelle le ou les émulgateurs appartiennent à l'une des classes ci-après :
   esters d'acides gras et de sorbitol
   esters d'acides gras et de mannitol
   esters d'acides gras et de saccharose
   esters d'acides gras et de glycérol
   un quelconque des esters ci-dessus condensés avec de l'oxyde d'éthylène et/ou de propylène
   acide gras condensé avec de l'oxyde d'éthylène et/ou de propylène
   alcool gras condensé avec de l'oxyde d'éthylène et/ou de propylène
   glycéro phospholipide.

3. Composition selon l'une quelconque des revendications 1 ou 2, caractérisée en ce que les acides gras utilisés pour les synthèses des émulgateurs possèdent de 12 à 22 atomes de carbone et en particulier sont des acides oléique, stéarique ou ricinoléique.

4. Composition selon l'une quelconque des revendications 1 à 3, caractérisée en ce que l'un des émulgateurs utilisé est un oléate de mannitol ou d'anhydromannitol modifié ou non par greffage de fonctions hydrophiles telles que groupement carboxylique, amine, amide, alcool, polyol, éther-oxyde.

5. Composition selon l'une quelconque des revendications 1 à 4, caractérisée par le fait que l'huile utilisée est une huile minérale ou un hydrocarbure de synthèse, liquide à 4°C et ayant une viscosité inférieure à 100 mPas à 40°C.

6. Composition selon l'une quelconque des revendications 1 à 4, caractérisée par le fait que l'huile utilisée est une huile ou une cire liquide métabolisable d'origine végétale, vierge ou raffinée.

7. Composition selon l'une quelconque des revendications 1 à 4, caractérisée par le fait que l'huile utilisée est une huile ou une cire d'origine animale, en particulier de poisson.

8. Composition selon l'une quelconque des revendications 1 à 7, caractérisée par le fait que l'huile utilisée est un mélange de deux ou plusieurs des huiles décrites dans les revendications 5 à 7.

9. Adjuvants huileux, liquides homogènes contenant au moins l'un des émulgateurs et huile décrits dans les revendications 2 à 8 et permettant d'obtenir, par agitation avec une phase aqueuse contenant un principe actif ou un antigène, une émulsion stable, de viscosité inférieure à 300 mPas fluide, de type E/H/E caractérisés par un point d'inversion compris entre 25 et 45°C.

10. Procédé pour la préparation de compositions injectables selon l'une quelconque des revendications 1 à 9 consistant à :
    - mélanger la ou les huiles et le ou les émulgateurs
    - stériliser cette phase par filtration, autoclavage ou tout autre moyen connu
    - chauffer cette phase entre 20 et 40°C, de préférence entre 30 et 35°C
    - préparer une solution aqueuse contenant les principes actifs ou les antigènes, stériliser cette solution aqueuse par tout moyen de stérilisation approprié
    - chauffer la solution aqueuse à la même température que la phase huileuse
    - ajouter la solution aqueuse dans la phase huileuse sous agitation non cisaillante.

**Patentansprüche**

1. Mehrphasige injizierbare Emulsionen vom Wasser/Öl/Wasser-Typ, die als Impfstoffe oder Träger von Wirkstoffen in der Veterinär- und Humanmedizin verwendbar sind, nur pharmazeutisch akzeptable Bestandteile enthalten, bei Lagerung bei 4 °C mindestens 12 Monate stabil sind, eine Viskosität von wenigen als 300 mPa.s besitzen und mindestens folgende Bestandteile enthalten:
   - Ein öliges Adjuvans, das besteht aus
      . einer nicht mit Wasser mischbaren Ölphase, die 20 bis 70 Gew.-% der Formulierung darstellt ;
      . einem Emulgatorsystem, das so ausgewählt ist, daß der Inversionspunkt der erhaltenen Emulsion zwischen 25 und 45 °C liegt, und das 2 bis 10 Gew.-% der Formulierung ausmacht;
   - einer wässerigen Phase, welche die Antigene oder Wirkstoffe enthält und 20 bis 78 Gew.-% der Formulierung darstellt,
   wobei die Emulsionen in einem einzigen Mischschritt erhalten sind.

2. Zusammensetzung nach Anspruch 1, bei welcher der oder die Emulgatoren einer der folgenden Klassen angehören:
   Ester von Fettsäuren mit Sorbit,
   Ester von Fettsäuren mit Mannit,
   Ester von Fettsäuren mit Saccharose,
   Ester von Fettsäuren mit Glycerin,
   Kondensationsprodukte eines der vorgenannten Ester mit Ethylenoxid und/oder Propylenoxid,
   Kondensationsprodukte von Fettsäuren mit Ethylenoxid und/oder Propylenoxid,
   Kondensationsprodukte von Fettalkoholen mit Ethylenoxid und/oder Propylenoxid,
   Glycerophospholipide.

3. Zusammensetzung nach Anspruch 1 oder 2, dadurch gekennzeichnet, daß die zur Synthese der Emulgatoren verwendeten Fettsäuren 12 bis 22 C-Atome besitzen und insbesondere Ölsäure, Stearinsäure oder Ricinoleinsäure darstellen .

4. Zusammensetzung nach einem der Ansprüche 1 bis 3, dadurch gekennzeichnet, daß einer der verwendeten Emulgatoren ein Mannit- oder Anhydromannitoleat ist, das ggfs. durch Aufpfropfen hydrophiler Funktionen wie Carboxylgruppen, Aminogruppen, Amidogruppen, Alkoholgruppen, Polyolgruppen oder Etheroxidgruppen modifiziert ist.

5. Zusammensetzung nach einem der Ansprüche 1 bis 4, dadurch gekennzeichnet, daß das verwendete Öl ein Mineralöl oder ein synthetischer Kohlenwasserstoff ist, bei 4 °C flüssig ist und bei 40 °C eine Viskosität von weniger als 100 mPa.s aufweist.

6. Zusammensetzung nach einem der Ansprüche 1 bis 4, dadurch gekennzeichnet, daß das verwendete Öl ein flüssiges, metabolisierbares Öl oder Wachs pflanzlichen Ursprungs ist, das in nichtraffinierter Form oder in raffinierter Form vorliegt.

7. Zusammensetzung nach einem der Ansprüche 1 bis 4, dadurch gekennzeichnet, daß das verwendete Öl ein Öl oder ein Wachs tierischen Ursprungs ist, insbesondere ein Fischöl oder Fischwachs.

8. Zusamnensetzung nach einem der Ansprüche 1 bis 7, dadurch gekennzeichnet, daß das verwendete Öl ein Gemisch von 2 oder mehr Ölen ist, wie sie in den Ansprüchen 5 bis 7 beschrieben sind.

9. Flüssige, homogene ölige Adjuvantien, die mindestens einen der Emulgatoren und Öle enthalten, die in den Ansprüchen 2 bis 8 beschrieben sind, und es erlauben, durch Rühren mit einer wässerigen Phase, die einen Wirkstoff oder ein Antigen enthält, eine stabile, flüssige Emulsion vom Wasser/Öl/Wasser-Typ und einer Viskosität von weniger als 300 mPa.s herzustellen, gekennzeichnet durch einen Inversionspunkt zwischen 25 und 45 °C.

10. Verfahren zur Herstellung der injizierbaren ZusammenSetzungen nach einem der Ansprüche 1 bis 9, das in folgenden Schritten besteht:
   - Mischen des oder der Öle und des oder der Emulgatoren,
   - Sterilisieren dieser Phase durch Filtration, Autoklavieren oder in einer beliebigen anderen, bekannten Weise,

- Erhitzen dieser Phase auf eine Temperatur zwischen 20 und 40 °C, vorzugsweise zwischen 30 und 35 °C, Herstellen einer wässerigen Lösung, welche die Wirkstoffe oder die Antigene enthält, Sterilisieren dieser wässerigen Lösung in beliebiger, geeigneter Weise,
- Erhitzen der wässerigen Lösung auf die gleiche Temperatur wie die Ölphase und
- Zugabe der wässerigen Lösung zur Ölphase unter Rühren ohne Scherbeanspruchung.


## Revendications

1. Injectable multi-phase emulsions of W/O/W type, usable as vaccines, or as vehicles for active substances in human or veterinary medecine, pharmaceutically acceptable in each one of their constituents, stable for at least 12 months in storage at 4°C, of viscosity less than 300 mPas and comprising at least the following constituents:
   - an oily adjuvant composed of:
     . a water immiscible oily phase representing between 20 and 70% of the formula,
     . an emulsifying system selected so that the inversion point of the resulting emulsion ranges between 25 and 45° and representing between 2 and 10% of the formula,
   - an aqueous phase containing the antigens or active substances and representing 20 to 78% of the formula and obtained in a single mixing operation.

2. Composition according to claim 1, in which the emulsifier or emulsifiers belong to one of the following classes:
   esters of fatty acids and of sorbitol
   esters of fatty acids and of mannitol
   esters of fatty acids and of saccharose
   esters of fatty acids and of glycerol
   any one of the aforesaid esters condensed with ethylene and/or propylene oxide
   fatty acid condensed with ethylene and/or propylene oxide fatty alcohol condensed with ethylene and/or propylene oxide
   glycero-phospholipid.

3. Composition according to any one of claims 1 or 2, characterized in that the fatty acids used for synthesizing the emulsifiers have between 12 and 22 carbon atoms and are in particular, oleic, stearic or ricinoleic acids.

4. Composition according to any one of claims 1 to 3, characterized in that one of the emulsifiers used is an oleate of mannitol or of anhydromannitol, whether or not modified by grafting or hydrophilic functions such as a carboxylic, amine, amide, alcohol, polyol, ether-oxide group.

5. Composition according to any one of claims 1 to 4, characterized by the fact that the oil used is a mineral oil or a synthetic hydrocarbon, which is liquid at 4°C and has a viscosity lower than 100 mPas at 40°C.

6. Composition according to any one of claims 1 to 4, characterized by the fact that the oil used is a metabolisable liquid oil or wax of vegetable origin, a virgin or refined oil.

7. Composition according to any one of claims 1 to 4, characterized by the fact that the oil used is an oil or wax of animal origin, principally fish.

8. Composition according to any one of claims 1 to 7, characterized by the fact that the oil used is a mixture of two or more of the oils described in claims 5 to 7.

9. Oily, liquid and homogeneous adjuvants containing at least one of the emulsifiers and oil described in claims 2 to 8 and permitting the preparation of a stable emulsion, by stirring with an aqeuous phase containing an active principle or an antigen, which emulsion has a viscosity lower than 300 mPas, fluid and of W/O/W type, said adjuvants having an inversion point ranging between 25 and 45°C.

10. Process for the preparation of the injectable compositions according to any one of claims 1 to 9, consisting in:

- mixing the oil or oils and the emulsifier or emulsifiers,
- sterilizing this phase by filtering, autoclaving or any other know means,
- heating this phase to between 20 and 40°C, and preferably between 30 and 35°C,
- preparing an aqueous solution containing the active substances or the antigens, sterilizing said aqueous solution by any suitable sterilizing means,
- heating the aqueous solution to the same temperature as the oily phase,
- adding the aqueous solution to the oily phase under non-splitting stirring.